# EUROPÄISCHE PATENTANMELDUNG

(11) **EP 0 992 286 A1**
(43) Veröffentlichungstag der Anmeldung: **12.04.2000**
(21) Anmeldenummer: 99119065.3
(22) Anmeldetag: 30.09.1999
(51) Int. Cl.: B01J 23/04, C07C 2/72, C07C 5/25, C07C 2/24, C07C 209/60

(54) **Herstellung eines basischen Katalysators unter Vermeidung hoher Temperaturen**

(30) Priorität: 01.10.1998 DE 19845293
(71) Anmelder: BASF AKTIENGESELLSCHAFT, 67056 Ludwigshafen (DE)
(72) Erfinder: Steinbrenner, Ulrich, Dr., 67063 Ludwigshafen (DE); Gehrer, Eugen, Dr., 67069 Ludwigshafen (DE)
(74) Vertreter: Isenbruck, Günter, Dr.

(57) **Zusammenfassung**

Katalysator, enthaltend mindestens ein Alkalimetall und mindestens einen metallischen oder halbmetallischen Promotor, ausgewählt aus der Gruppe bestehend aus Ca, Sr, Ba, Ag, Au, Zn, Cd, Hg, In, Tl, Sn, As, Sb und Bi, auf einem Träger, der mit einer oder mehreren Verbindungen eines Alkalimetalls und/oder Erdalkalimetalls dotiert sein kann, wobei das Gewichtsverhältnis Alkalimetall/Träger 0,01 bis 5, das Gewichtsverhältnis Promotor/Alkalimetall 0,0001 bis 5 und bei Vorliegen einer Dotierung das Gewichtsverhältnis Dotierung/Träger 0,01 bis 5 beträgt.

## Beschreibung

Die Erfindung betrifft einen Katalysator, ein Verfahren zu seiner Herstellung, seine Verwendung in stark basisch katalysierten Reaktionen sowie ein Verfahren zur Seitenkettenalkylierung oder Seitenkettenalkenylierung von alkylaromatischen Verbindungen mit Olefinen oder Diolefinen.

Die Seitenkettenalkylierung von aromatischen Verbindungen, die ein azides Proton in der α-Position der Seitenkette tragen, in Gegenwart von basischen Katalysatoren ist bekannt. Bekannte basische Katalysatoren bestehen aus einem Träger, der mit einer oder mehreren Verbindungen eines Alkalimetalls und/oder Erdalkalimetalls dotiert sein kann, und einem Alkalimetall oder einer Alkalimetallegierung als Aktivkomponente.

In der EP-B-0 439 679 ist ein Verfahren zur Alkylierung von alkylaromatischen Kohlenwasserstoffen beschrieben. Die Umsetzung erfolgt in Gegenwart eines Katalysators aus aktiviertem Aluminiumoxid, das mit Magnesiumhydroxid und Kaliummetall dotiert ist. Anstelle von Magnesiumhydroxid kommen auch Calciumhydroxid, Bariumhydroxid oder Magnesiumoxid zum Einsatz. Auch eine Imprägnierung mit Kaliumhydrid ist beschrieben.

US 4,914,250 betrifft ein Verfahren zur Seitenkettenalkylierung von Aromaten. Als Katalysator wird dabei Diatomeenerde eingesetzt, die im Reaktionsgemisch neben Kalium oder NaK und Spuren von Wasser vorliegt.

US 4,922,054 betrifft ebenfalls ein Verfahren zur Seitenkettenalkylierung von Aromaten, bei dem ebenfalls als Katalysator die Diatomeenerde eingesetzt wird, die im Reaktionsgemisch neben NaK und Kaliumoxid vorliegt. Anstelle von Kaliumoxid wird auch Rubidiumoxid verwendet. Anstelle von NaK wird auch Kaliummetall eingesetzt.

JP-A2-05163171 betrifft die Herstellung von Alkenylbenzol und seinen Derivaten. Der verwendete Katalysator umfaßt ein Alkalimetall und ein Kaliumcarbonatsalz und/oder KOH, die in Gegenwart eines Olefins und/oder Diolefins dispergiert werden. Vorzugsweise wird als Alkalimetall Natriummetall und als Kaliumcarbonatsalz K₂CO₃, KHCO₃ oder KNaCO3 eingesetzt.

Das Aufbringen des Alkalimetalls bzw. der Alkalimetallegierung als Aktivkomponente auf den dotierten Katalysatorträger gelingt bei Temperaturen deutlich unter 300°C nur schlecht, da das Metall den Träger dann kaum benetzt. Die so erhaltenen Katalysatoren weisen eine nur geringe Aktivität auf, d. h. die Raum-Zeit-Ausbeute ist sehr klein. Andererseits ist es wünschenswert, die Katalysatorpräparation schon in dem Reaktionsbehälter durchzuführen, in dem später die Seitenkettenalkylierung durchgeführt wird. Dadurch kann ein Umfüllen des luftempfindlichen, pyrophoren Katalysators entfallen. Viele Reaktionsapparate werden jedoch mit einer Dampf- oder Ölheizung betrieben und sind nicht für Temperaturen um 300°C ausgelegt. Auch kommt es bei Temperaturen um 300°C in nicht unwesentlichem Ausmaß zur Verdampfung von Alkalimetall, was zu Korrosionsproblemen führen kann.

Aufgabe der vorliegenden Erfindung ist es, einen basischen Katalysator aus einem Alkalimetall als Aktivkomponente auf einem Träger zur Seitenkettenalkylierung alkylaromatischer Verbindungen bereit zu stellen, der bei Temperaturen deutlich unter 300°C präpariert werden kann und sich durch eine hohe Aktivität auszeichnet.

Die Aufgabe wird erfindungsgemäß gelöst durch einen Katalysator, enthaltend mindestens ein Alkalimetall und mindestens einen metallischen oder halbmetallischen Promotor, ausgewählt aus der Gruppe bestehend aus Ca, Sr, Ba, Ag, Au, Zn, Cd, Hg, In, Tl, Sn, As, Sb und Bi, auf einem Träger, der mit einer oder mehreren Verbindungen eines Alkalimetalls und/oder Erdalkalimetalls dotiert sein kann, wobei das Gewichtsverhältnis Alkalimetall/Träger 0,01 bis 5, das Gewichtsverhältnis Promotor/Alkalimetall 0,0001 bis 5 und bei Vorliegen einer Dotierung das Gewichtsverhältnis Dotierung/Träger 0,01 bis 5 beträgt.

Ferner wird die Aufgabe gelöst durch Verwendung dieses Katalysators in stark basisch katalysierten Reaktionen, vorzugsweise zur Seitenkettenalkylierung oder Seitenkettenalkenylierung von alkylaromatischen Verbindungen mit Olefinen oder zur Doppelbindungsisomerisierung von Olefinen, zur Dimerisierung von Olefinen oder zur basischen Aminierung von Olefinen.

Die Aufgabe wird ferner beispielhaft gelöst durch Bereitstellung eines Verfahrens zur Seitenkettenalkylierung oder Seitenkettenalkenylierung von alkylaromatischen Verbindungen durch Umsetzung mit Olefinen oder Diolefinen, wobei die Umsetzung in Gegenwart eines vorstehend definierten Katalysators durchgeführt wird.

Durch den Zusatz eines metallischen oder halbmetallischen Promotors, der sich in dem flüssigen Alkalimetall bei Temperaturen unterhalb 300°C merklich löst, werden die Benetzungseigenschaften des flüssigen Alkalimetalls deutlich verbessert.

Das Gewichtsverhältnis Alkalimetall/Träger beträgt dabei vorzugsweise 0,01 bis 2, besonders bevorzugt 0,01 bis 1. Das Alkalimetall ist dabei vorzugsweise Natrium oder Kalium, insbesondere Natrium. Auch Gemische mehrerer Alkalimetalle können eingesetzt werden. Bevorzugtes Gemisch ist eine Natrium/Kalium-Legierung.

Das Gewichtsverhältnis Promotor/Alkalimetall beträgt vorzugsweise 0,0001 bis 1, besonders bevorzugt 0,0001 bis 0,3, insbesondere 0,01 bis 0,1. Bevorzugte Promotoren sind Ca, Sr, Ba, Zn, In, Sn, Sb, besonders bevorzugt sind Ca, Sr, Ba und Zn. Es können auch Gemische mehrerer Promotoren eingesetzt werden.

Katalysatorträger sind übliche Träger wie Al₂O₃, La₂O₃, ZrO₂, Graphit, Diatomäenerde, Spinelle, Inversspinelle, Erdalkalioxide, Alkalicarbonate, Erdalkalicarbonate, Titanate, Zirkonaten und Hafnate.

Der Träger kann ferner mit mindestens einer Verbindung eines Alkalimetalls und/oder Erdalkalimetalls im Gewichtsverhältnis Dotierung/Träger von 0,01 bis 5, vorzugsweise 0,01 bis 2, insbesondere 0,01 bis 1 dotiert sein. Vorzugsweise ist der Katalysator so dotiert. Die Dotierung der Träger erfolgt dabei vorzugsweise mit löslichen Verbindungen der Alkalimetalle und/oder Erdalkalimetalle, wie den Oxiden, Hydroxiden, Carbonaten, Formiaten, Acetaten, Oxalaten und/oder Hydriden. Bevorzugt werden die Hydroxide oder Carbonate, besonders bevorzugt K₂CO₃ und/oder KOH eingesetzt.

Die Katalysatoren werden hergestellt durch
- Aufbringen mindestens eines Alkalimetalls und mindestens eines metallischen oder halbmetallischen Promotors auf den Träger durch Trägerung einer schmelzflüssigen Lösung des Promotors in dem Alkalimetall,
- wobei der Träger gegebenenfalls vorher dotiert wurde durch Imprägnieren oder Tränken mit einer Lösung mindestens einer Verbindung eines Alkalimetalls und/oder Erdalkalimetalls, Trocknen und Calcinieren des dotierten Trägers.

Das Alkalimetall und der metallische oder halbmetallische Promotor werden durch Trägerung im schmelzflüssigen Zustand auf den Träger aufgebracht. Die Trägerung erfolgt dabei bei einer Temperatur bevorzugt von 80 bis 400°C, besonders bevorzugt von 100 bis 200°C. Dazu wird die entsprechende Menge des Alkalimetalls als Strang oder Block zusammen mit der entsprechenden Menge des Promotors in elementarer Form zum Träger gegeben und unter Erwärmen mit ihm vermischt. Es kann auch eine Legierung, eine intermetallische Phase oder Verbindung des Alkalimetalls mit dem Promotor als Strang, Block, Grieß oder Pulver zugegeben werden. Beim Vermischen der schmelzflüssigen Lösung des Promotors in dem Alkalimetall mit dem Träger erfolgt eine feine Verteilung des Alkalimetalls auf dem Träger. Die Trägerung der Alkalimetalle kann im Vakuum, unter Inertgasatmosphäre (He, N₂, Ar usw.) oder unter Reaktivgasatmosphäre (H₂, CO₂, CO) stattfinden.

Die Dotierung des Trägers erfolgt in an sich bekannter Weise durch Imprägnierung oder Tränkung und anschließende Calcinierung bei Temperaturen im Bereich von 100 bis 1500°C, vorzugsweise 250 bis 1000°C, besonders bevorzugt 250 bis 350°C. Dabei kann die Imprägnierung oder Tränkung mit einer Lösung der Verbindung des Alkalimetalls und/oder Erdalkalimetalls in einem beliebigen geeigneten Lösungsmittel erfolgen. Vorzugsweise werden wäßrige Lösungen eingesetzt, wobei nach dem Imprägnieren oder Tränken das Wasser durch Trocknen des imprägnierten Trägers entfernt wird. Es kann auch ohne vorherige Trocknung calciniert werden, wobei zu Beginn der Calcinierung das Lösungsmittel entweicht. Die Calcinierung des dotierten Trägers kann unter vermindertem Druck, unter Normaldruck oder unter erhöhtem Druck durchgeführt werden. Sie kann dabei sowohl in sauerstoffhaltiger Atmosphäre, als auch in Inertgasatmosphäre, wie unter Helium, Stickstoff oder Argon, oder unter Reaktivgasatmosphäre, wie unter Wasserstoff, Ammoniak, Kohlendioxid oder Kohlenmonoxid stattfinden.

Die Katalysatoren werden in stark basisch katalysierten Reaktionen, vorzugsweise zur Seitenkettenalkylierung oder Seitenkettenalkenylierung von alkylaromatischen Verbindungen mit Olefinen oder Diolefinen, zur Doppelbindungsisomerisierung von Olefinen, zur Dimerisierung von Olefinen oder zur basischen Aminierung von Olefinen eingesetzt.

Dabei wird die Umsetzung im allgemeinen bei einer Temperatur von -50 bis 400°C, vorzugsweise bei einer Temperatur von -20 bis 300°C, besonders bevorzugt 80 bis 250°C, insbesondere 100 bis 220°C und einem Druck von vorzugsweise 0,1 bis 200, besonders bevorzugt 1 bis 150, insbesondere 1 bis 100 bar durchgeführt.

Als alkylaromatische Verbindungen können dabei alle geeigneten Alkylaromaten eingesetzt werden. Sie können als aromatischen Kern beispielsweise einen Benzol- oder Naphthalinkern aufweisen. Auch Reste, in denen mehrere der Ringstrukturen miteinander verknüpft sind, können eingesetzt werden. Die Ringstrukturen weisen in α-Position der Seitenkette ein azides Wasserstoffatom auf. Vorzugsweise weisen sie mindestens einen Alkylrest auf, der an die cyclische Struktur gebunden ist. Die Alkylreste können dabei eine beliebige Länge aufweisen und durch weitere Substituenten substituiert sein. Vorzugsweise werden als alkylaromatische Verbindungen Benzole, die durch 1 bis 6, vorzugsweise 1 bis 3, insbesondere 1 bis 2 C₁₋₂₀-, vorzugsweise C₁₋₃-Alkylreste substituiert sind, eingesetzt.

Die Olefine weisen vorzugsweise 2 bis 20, besonders bevorzugt 2 bis 10, insbesondere 2 bis 5 C-Atome auf. Vorzugsweise werden Ethen, Propen, 1-Buten, 2-Buten, Isobuten, 1-Penten, 2-Penten, 2-Methyl-1-buten, 2-Methyl-2-buten und/oder 3-Methyl-1-buten eingesetzt. Besonders bevorzugt sind Ethen und Propen. Die Diolefine weisen vorzugsweise 4 bis 20, besonders bevorzugt 4 bis 10, insbesondere 4 bis 6 C-Atome auf. Besonders bevorzugt werden Butadien und/oder Isopren eingesetzt.

Olefine, die mit dem erfindungsgemäßen Katalysator isomerisiert oder dimerisiert werden können, sind beispielsweise die vorstehend genannten Olefine. Olefine, die mit den erfindungsgemäßen Katalysatoren aminiert werden können, sind insbesondere Ethen oder konjugierte Diene wie Butadien oder Isobuten, wobei als Amine vorzugsweise Ammoniak, Diethylamin, Ethylamin, Diisopropylamin oder Pyrrolidin eingesetzt werden.

Besonders bevorzugt sind die Umsetzung von Toluol mit Ethen oder Propen zu Propylbenzol oder Isobutylbenzol, die Umsetzung von Cumol mit Ethen zu tert.-Amylbenzol und die Umsetzung von Xylolen mit Butadien zu 5-Tolylpentenen sowie die Umsetzung von p-Xylol mit 1-Buten oder 2-Buten zu 1-Tolyl-2-Methylbutan.

Die Umsetzung kann diskontinuierlich oder vorzugsweise kontinuierlich in der Flüssig- oder Gasphase, bevorzugt in der Flüssigphase durchgeführt werden. Dabei können die bekannten Vorrichtungen zur Durchführung des Verfahrens eingesetzt werden.

Nachstehend wird die Erfindung anhand von Beispielen weiter erläutert.

### Beispiele

### Herstellungsbeispiele

Am Ende jedes Beispiels sind die Elementaranalysen pro 100 g des dotierten Trägers vor der Aufbringung des Alkalimetalls in Klammern angegeben.

### Katalysator A (Vergleich)

10 g γ-Al₂O₃ wurden mit 1 g K₂CO₃ (gelöst in H₂O) imprägniert. Die Suspension wurde zur Trockene eingedampft und das so erhaltene Pulver bei 300°C im Argonstrom unter Rühren 15 Stunden lang calciniert. Zu diesem Pulver wurden 1 g metallischen Natrium zugefügt und bei 160°C 2 Stunden lang dispergiert.

### Katalysator B

Herstellung wie Katalysator A, wobei anstelle von Natrium 1 g einer Legierung von 49 Gew.-% Barium und 51 Gew.-% Natrium eingesetzt wurden.

### Katalysator C

Herstellung wie Katalysator A, wobei anstelle von Natrium 1 g einer Legierung von 6 Gew.-% Barium und 94 Gew.-% Natrium eingesetzt wurden.

### Katalysator D

Herstellung wie Katalysator A, wobei zusätzlich 0,1 g Sn zugegeben wurden.

### Katalysator E

Herstellung wie Katalysator A, wobei zusätzlich 0,1 g Pb zugegeben wurden.

### Katalysator F

Herstellung wie Katalysator A, wobei zusätzlich 0,1 g Tl zugegeben wurden.

### Katalysator G

Herstellung wie Katalysator A, wobei zusätzlich 0,1 g Ca zugegeben wurden.

### Katalysator H

Herstellung wie Katalysator A, wobei zusätzlich 0,1 g Sr zugegeben wurden.

### Verfahrensbeispiele

### Vergleichsbeispiel V1

10 g Katalysator A wurden mit 85 g Toluol in einem druckfestem Reaktionsgefäß vorgelegt. Nach Zugabe von 20 g Propen wurde das Reaktionsgefäß auf 160°C aufgeheizt und die Reaktionsuspension anschließend 12 Stunden lang gerührt. Die Ergebnisse sind in der Tabelle aufgeführt.

### Beispiele 1 bis 7

10 g der Katalysatoren B bis H wurdenjeweils mit 85 g Toluol in einem druckfestem Reaktionsgefäß vorgelegt. Nach Zugabe von 20 g Propen wurde das Reaktionsgefäß auf 160°C aufgeheizt und die Reaktionssuspension anschließend 12 Stunden lang gerührt. Die Ergebnisse sind in der Tabelle aufgeführt (alle Angaben in Mol-%).

| Beispiel | Katalysator | U_{Propen} | S_{iBB}* | S_{MP} | U_{Toluol} | S_{iBB}** |
|---|---|---|---|---|---|---|
| V1 | A | 23 | 51 | 44 | 6 | 91 |
| 1 | B | 37 | 56 | 39 | 11 | 92 |
| 2 | C | 32 | 61 | 32 | 11 | 90 |
| 3 | D | 33 | 57 | 38 | 10 | 91 |
| 4 | E | 50 | 58 | 38 | 16 | 93 |
| 5 | F | 52 | 55 | 40 | 23 | 93 |
| 6 | G | 51 | 59 | 37 | 16 | 93 |
| 7 | H | 64 | 57 | 38 | 20 | 93 |
| U_{Propen} = Propen-Umsatz [Mol-%] S_{iBB}* = Selektivität für Isobutylbenzol bezüglich Propen [Mol-%] S_{MP} = Selektivität für Methylpenten bezüglich Propen [Mol-%] S_{iBB}** = Selektivität für Isobutylbenzol bezüglch Toluol [Mol-%] U_{Toluol} = Toluol-Umsatz [Mol-%] | | | | | | |

## Patentansprüche

1. Katalysator, enthaltend mindestens ein Alkalimetall und mindestens einen metallischen oder halbmetallischen Promotor, ausgewählt aus der Gruppe bestehend aus Ca, Sr, Ba, Ag, Au, Zn, Cd, Hg, In, Tl, Sn, As, Sb und Bi, auf einem Träger, der mit einer oder mehreren Verbindungen eines Alkalimetalls und/oder Erdalkalimetalls dotiert sein kann, wobei das Gewichtsverhältnis Alkalimetall/Träger 0,01 bis 5, das Gewichtsverhältnis Promotor/Alkalimetall 0,0001 bis 5 und bei Vorliegen einer Dotierung das Gewichtsverhältnis Dotierung/Träger 0,01 bis 5 beträgt.

2. Katalysator nach Anspruch 1, dadurch gekennzeichnet, daß der Träger ausgewählt ist aus der Gruppe bestehend aus Al₂O₃, La₂O₃, ZrO₂, Graphit, Diatomäenerde, Spinellen, Inversspinellen, Erdalkalioxiden, Alkalicarbonaten, Erdalkalicarbonaten, Titanaten, Zirkonaten und Hafnaten.

3. Katalysator nach Anspruch 1 oder 2, dadurch gekennzeichnet, daß der mit mindestens einem Hydroxid oder Carbonat eines Alkalimetalls und/oder Erdalkalimetalls dotiert ist.

4. Verfahren zur Herstellung von Katalysatoren, wie sie in einem der Ansprüche 1 bis 3 definiert sind, durch
- Aufbringen mindestens eines Alkalimetalls und mindestens eines metallischen oder halbmetallischen Promotors auf den Träger durch Trägerung einer schmelzflüssigen Lösung des Promotors in dem Alkalimetall,
- wobei der Träger gegebenenfalls vorher dotiert wurde durch Imprägnieren oder Tränken mit einer Lösung mindestens einer Verbindung eines Alkalimetalls und/oder Erdalkalimetalls, Trocknen und Calcinieren des dotierten Trägers.

5. Verwendung eines Katalysators, wie er in einem der Ansprüche 1 bis 3 definiert ist, zur Seitenkettenalkylierung oder Seitenkettenalkenylierung von alkylaromatischen Verbindungen mit Olefinen oder Diolefinen, zur Doppelbindungsisomerisierung von Olefinen, zur Dimerisierung von Olefinen oder zurbasischen Aminierung von Olefinen.

6. Verfahren zur Seitenkettenalkylierung oder Seitenkettenalkenylierung von alkylaromatischen Verbindungen durch Umsetzung mit Olefinen oder Diolefinen, dadurch gekennzeichnet, daß die Umsetzung in Gegenwart eines Katalysators durchgeführt wird, wie er in einem der Ansprüche 1 bis 3 definiert ist.

7. Verfahren nach Anspruch 6, dadurch gekennzeichnet, daß die Umsetzung bei einer Temperatur im Bereich von -50 bis 400°C und einem Druck im Bereich von 0,1 bis 200 bar durchgeführt wird.

8. Verfahren nach einem der Ansprüche 6 oder 7, dadurch gekennzeichnet, daß als alkylaromatische Verbindungen Benzole, die durch 1 bis 6 C₁₋₂₀-Alkylreste substituiert sind, und/oder Naphthaline, die durch 1 bis 10 C₁₋₂₀ Alkylreste substituiert sind, eingesetzt werden.

9. Verfahren nach einem der Ansprüche 6 bis 8, dadurch gekennzeichnet, daß als Olefine Ethen, Propen, 1-Buten, 2-Buten, Isobuten, 1-Penten, 2-Penten, 2-Methyl-1-buten, 2-Methyl-2-buten und/oder 3-Methyl-1-buten, und als Diolefine Butadien und/oder Isopren eingesetzt werden.

10. Verfahren nach einem der Ansprüche 6 bis 9, dadurch gekennzeichnet, daß es in der Flüssig- oder Gasphase kontinuierlich durchgeführt wird.
